# EUROPÄISCHE PATENTSCHRIFT

(11) **EP 1 611 862 B1**
(45) Veröffentlichungstag und Bekanntmachung des Hinweises auf die Patenterteilung: **04.04.2007**
(21) Anmeldenummer: 04015307.4
(22) Anmeldetag: 30.06.2004
(51) Int. Cl.: A61B 19/00

(54) **Nicht-invasive Navigationsreferenz-Fixierung**
Non-invasive fixation of a navigation reference
Fixation non-invasive de référence de navigation

(43) Veröffentlichungstag der Anmeldung: 04.01.2006
(73) Patentinhaber: BrainLAB AG, 85551 Kirchheim/Heimstetten (DE)
(72) Erfinder: Schaffrath, Claus, 81377 München (DE); Kleinszig, Gerhard, 85656 Buch (DE); Schenkel, Christoph, 82178 Puchheim (DE)
(74) Vertreter: Schwabe - Sandmair - Marx

(56) Entgegenhaltungen:
- DE-A- 10 206 166
- US-A- 3 762 404
- US-A1- 2002 188 194
- US-B1- 6 241 735

## Beschreibung

Die vorliegende Erfindung betrifft ein Fixierungssystem zum Fixieren einer Navigations-Referenzanordnung für die bildunterstützte medizinische Behandlung gegenüber einem Patienten. Allgemeiner ausgedrückt betrifft die Erfindung das Gebiet der nicht-invasiven Fixierung einer Navigationsreferenz, die durch das Ortungssystem eines medizinischen Navigationssystems positionell bestimmt und verfolgt werden kann, um dadurch einen Patienten, beispielsweise die innere Struktur seiner Körperteile, mit Navigationsunterstützung behandeln zu können. Vorab erfasste Daten über die Körperstruktur des Patienten können sichtbar im richtigen Positionsverhältnis zu verwendeten Instrumenten oder Behandlungseinrichtungen dargestellt werden, weil mittels der fest positionell gegenüber dem Patientenkörperteil angeordneten Referenz zu jedem Zeitpunkt die Lage der Körperstrukturen und die dazu im Verhältnis stehende Lage der Instrumente bzw. Behandlungseinrichtungen bekannt ist. Da die Daten sichtbar auf Navigationsbildschirmen dargestellt werden können, kann minimalinvasiv operiert werden.

Insbesondere betrifft die vorliegende Erfindung das Gebiet der intraoperativen Navigation mit berührungsloser Registrierung der Referenz bzw. der Referenzanordnung, speziell für Anwendungen im spinalen Bereich.

Wie oben schon erwähnt, muss eine Patientenreferenz fest gegenüber dem zu behandelnden Körperteil angeordnet werden, um eine erfolgreiche Navigation sicher zu stellen. Dies wurde bisher beispielsweise im Bereich der Wirbelsäulenbehandlung dadurch erreicht, dass man die Referenzanordnung an einem freigelegten Knochenabschnitt befestigte und dann im Navigationssystem registrierte, also positionell zuordnete. Der Nachteil solcher Verfahren liegt darin, dass relativ große Hauteinschnitte und Gewebedurchschnitte durchzuführen sind, um den Zugang zu dem Knochenabschnitt (z.B. Wirbelkörper) zu gestatten, an welchem die Referenzanordnung zu befestigen ist. Insbesondere wenn der eigentliche Eingriff beispielsweise mit einem dünnen Instrument von außen durchgeführt werden könnte, entsteht dann das Problem, dass die Befestigung der Referenz, die eigentlich eine minimal invasive Behandlung garantieren soll, den invasivsten Eingriff der gesamten Behandlung darstellen würde.

Aus der DE 102 06 166 A1 ist eine Ortungsvorrichtung zur Bestimmung der Lage eines Patienten bekannt, wobei eine Vakuummatratze verwendet wird, um den Patienten zu lagern. Um die Vakuummatratze und den Patienten herum wird eine kastenartige Liege gesetzt, mit zwei Seitenwänden, die zu beiden Seiten des Patienten nach oben stehen und an denen Navigationsmarkierungen angeordnet sind. Auf diese Weise soll der Patient selbst immobilisiert und gegenüber den Markierungen fixiert werden. Nachteilig wirkt sich dabei aus, dass der Patient relativ weit von den Markierungen entfernt ist, was die Navigation ungenauer macht. Außerdem ist die vorgeschlagene kastenartige Anordnung sehr sperrig und schwer handhabbar, und sie ist bei der eigentlichen Behandlung im Wege.

Die DE 197 31 040 A1 beschreibt eine Vorrichtung und ein Verfahren zur Fixierung von Körperteilen, wobei ein Patient auf einer Auflagefläche positioniert und dann mit einer Folie überdeckt wird. Durch das Absaugen der Luft unter der Folie soll der Patient immobilisiert werden. Als ein Nebenaspekt wird noch beschrieben, dass Haltearme verwendet werden können, die einen Sockel unterhalb der Folie aufweisen, durch diese hindurchgehen und am anderen Ende an der Patientenliege befestigt sind. Die Haltearme sind mit Gelenken ausgeführt und sowohl am Patienten als auch an der Liege lediglich durch die mit Unterdruck abgesaugte Folie befestigt. Es wird vorgeschlagen, an diesen Haltearmen auch ein Markersystem anzubringen, jedoch kann die Befestigung durch die mit Unterdruck angelegte Folie keinen ausreichenden Halt bieten, um solche Markierungen so starr gegenüber dem Körper zu fixieren, dass eine ausreichende Genauigkeit bei der Navigation vorausgesetzt werden kann. Wegen der Gelenkigkeit der Haltearme und der labilen Fixierung mittels der Folie kann eine feste und fixierte Anordnung solcher Markierungen gegenüber den Körperteilen nicht garantiert werden.

Aus der US 2002/01818194 A1 ist ein chirurgisches Positionierungssystem mit Patientenmarkern und Redundanzmarkern an starren Patientenschalen bekannt.

Es ist die Aufgabe der vorliegenden Erfindung, einen Weg aufzuzeigen, wie eine Navigations-Referenzanordnung gegenüber einem Patienten bzw. dem zu behandelndem Körperteil auf nicht-invasive Weise fixiert werden kann, ohne dass bei der Genauigkeit der danach verwendeten Navigation Abstriche gemacht werden müssen, und ohne die Verwendung komplizierter oder sperriger Gerätschaften.

Diese Aufgabe wird erfindungsgemäß durch ein Fixierungssystem gemäß dem Anspruch 1 gelöst. Die Unteransprüche definieren bevorzugte Ausführungsformen der vorliegenden Erfindung.

Die Vorteile der vorliegenden Erfindung beruhen darauf, dass bei einem Fixierungssystem, bei dem die Referenzanordnung unter Mitwirkung einer Immobilisierungseinrichtung außerhalb des Patientenkörpers angeordnet, wird, die Immobilisierungseinrichtung eine Vakuummatratze oder eine Vakuummatratzen-Patientenumhüllung ist, und darauf, dass an oder in der Vakuummatratze bzw. Vakuummatratzen-Patientenumhüllung mindestens eine Verankerung für die Referenzanordnung vorgesehen ist. Gemäß der Erfindung wird die Referenzanordnung direkt an der Immobilisationseinrichtung selbst angebracht, so dass einerseits die Nähe der Referenzanordnung zu dem zu behandelnden Patientenkörperteil sichergestellt werden kann und andererseits keine möglicherweise beweglichen Teile zwischen dem Patienten und der Referenzanordnung selbst liegen. Die Genauigkeit der Navigation kann so sichergestellt werden. Weil die Immobilisierungseinrichtung eine Vakuummatratze oder eine Vakuummatratzen-Patientenumhüllung ist, die nach dem Evakuieren völlig starr wird, ist gerade eine solche Matratze ein geeigneter Ort, um die Referenzanordnung stabil und unbeweglich gegenüber dem Patienten zu fixieren. Aus diesem Grund genügt es auch, eine Verankerung für die Referenzanordnung vorzusehen, auf welcher dann die Referenzanordnung fixiert werden kann.

Bei einer bevorzugten Ausführungsform der vorliegenden Erfindung weist die Verankerung einen Sockel bzw. eine Sockelplatte auf (die Sockelplatte muss nicht notwendigerweise starr sein, sondern kann auch aus einem flexiblen Material sein, und bei der Evakuierung starr werden), der bzw. die bei der Evakuierung der Vakuummatratze oder der Vakuummatratzen-Patientenumhüllung gegenüber dieser in einer festen Position starr fixiert wird. Ein solcher Sockel bzw. eine solche Sockelplatte sorgt für einen ausreichenden Halt, um die Fixierung starr und stabil zu machen. Insbesondere kann der Sockel bzw. die Sockelplatte innerhalb des Füllmaterials der Vakuummatratze bzw. Vakuummatratzen-Patientenumhüllung eingebettet sein und eine Verbindung zur Referenzanordnung aufweisen, die durch eine gasdicht abgeschlossene Öffnung zur Außenseite ragt. Bei dieser Anordnung im Inneren des Matratzenmaterials ist gewährleistet, dass der Sockel bzw. die Sockelplatte nach der Evakuierung von allen Seiten unbeweglich gehalten wird.

Die Referenzanordnung kann einteilig mit der Verbindung und dem Sockel bzw. der Sockelplatte ausgebildet sein, es besteht aber auch die Möglichkeit, die Referenzanordnung als separates Bauteil auszubilden, so dass sie über einen Adapter auf die Verbindung gesetzt werden kann. Letztere Ausgestaltung gestattet eine sehr einfache Handhabung und schafft die Möglichkeit, die Referenzanordnung erst dann aufzusetzen, wenn sie benötigt wird, also beispielsweise nach der Immobilisierung des Patienten kurz vor oder während der Behanldung.

Einen besonderen Vorteil bietet eine Ausführungsform der vorliegenden Erfindung, bei der mehrere Verankerungen an beabstandeten Stellen der Vakuummatratze bzw. der Vakuummatratzen-Patientenumhüllung vorgesehen sind. Diese Ausbildung ermöglicht es nämlich, den Patienten mit der Vakuummatratze bzw. der Vakuummatratzen-Patientenumhüllung zu immobilisieren und dann an gerade geeigneten Verankerungen eine Referenzanordnung aufzusetzen, was beispielsweise durch eine Steck-, Schnapp-, Gewinde-, oder eine sonstige lösbare Verbindung geschehen kann. Eine geeignete Stelle ist meist diejenige, die möglichst nah an dem Patientenkörperteil ist, an dem operiert werden soll, und mit der Bereitstellung mehrerer Verankerungen an beabstandeten und voraussichtlich geeigneten Stellen, z.B. im Randbereich der Matratze bzw. Umhüllung, kann diesem Erfordernis erfolgreich Rechnung getragen werden; die so ausgestaltete Immobilisierungseinrichtung wird universell anwendbar.

Die Verwendung einer Vakuummatratze bzw. der Vakuummatratzen-Patientenumhüllung bietet außerdem noch einen weiteren wichtigen Vorteil. Solche Matratzeneinrichtungen können in der Form frei gestaltet werden, so dass es gemäß einem Aspekt der Erfindung auch möglich ist, mindestens eine Aussparung bzw. ein Durchgangsfenster vorzusehen, das den Zugang zu behandlungsbedürftigen Patientenkörperteilen gestattet. Es kann also einerseits eine gute Immobilisierung und eine feste Verankerung der Referenzanordnung, andererseits aber auch ein optimaler Zugang zu der zu behandelnden Patientenregion erzielt werden. Bei einer solchen Ausführungsform ist es von Vorteil, wenn die Verankerungen um die Peripherie des Durchgangsfensters herum angeordnet sind, um sie in die Nähe des Behandlungsortes zu bringen.

Eine weitere Ausführungsform der Erfindung sieht vor, dass die Vakuummatratzen-Patientenumhüllung so bemessen ist, dass sie den Rumpfbereich des Patienten umhüllen kann, wobei sie mit Hilfe von Schließen an ihren Randbereichen vorfixiert wird, bevor die endgültige Immobilisierung durch das Entlüften der Matratze erfolgt.

Die Erfindung wird im Weiteren anhand einer bevorzugten Ausführungsform näher erläutert. Die in dieser Beschreibung aufgeführten Merkmale der Erfindung können einzeln und in jedweder Kombination umgesetzt werden. In den beiliegenden Figuren zeigen:
- Figur 1: eine schematische Aufsicht von oben auf einen Patienten, der mit einer Vakuummatratzen-Umhüllung immobilisiert ist, welche ein erfindungsgemäßes Fixierungssystem aufweist;
- Figur 2: eine Ansicht des Schnittes A-A in Figur 1; und
- Figur 3: eine Referenzanordnung mit Sockel, die bei dem erfindungsgemäßen Fixierungssystem verwendet werden kann.

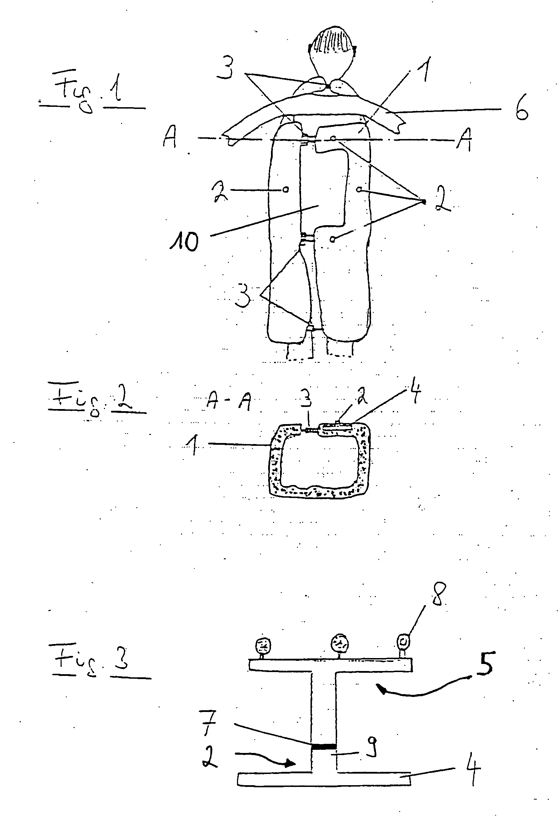

In der Figur 1 ist ein Patient in Bauchlage von oben gezeigt, an dem eine Operation im Rückenwirbelbereich durchgeführt werden soll. Der Patient wird mit Hilfe eines Bandes 6 auf seiner Auflage gehalten, jedoch muss im vorliegenden Fall damit nicht unbedingt eine völlig starre Fixierung des Patienten erfolgen. Dies liegt daran, dass der Patient von einer Vakuummatratzen-Patientenumhüllung eingeschlossen ist, die das Bezugszeichen 1 trägt und an der Verankerungen 2 für Referenzanordnungen vorgesehen sind. Der Patient kann sich deshalb nur zusammen mit der Umhüllung 1 bewegen, und weil sich dabei Referenzanordnungen in den Verankerungen 2 mitbewegen würden, wird die entsprechende Bewegung bei der Navigation berücksichtigt, und es entstehen keine Fehler. Es ist deshalb nicht notwendig, den Patienten vollständig auf seiner Auflage zu fixieren; wichtig ist lediglich, dass die Verankerungen und damit die Referenzanordnungen sich nicht gegenüber dem Patienten bewegen können, und dies wird durch die festsitzende Vakuummatratzen-Umhüllung gewährleistet. Der Patient hat deshalb wesentlich mehr Bequemlichkeit als bei einer vollständigen Immobilisierung.

Die Vakuummatratzen-Umhüllung 1 umschließt den Patienten im Rumpfbereich, und sie kann vor dem Absaugen der Luft aus ihrem Inneren mit den Schließen 3 vorbefestigt werden. Die Umhüllung 1 weist bei der dargestellten Ausführungsform ein Fenster 10 auf, durch welches der Zugang zum Operationsbereich gewährleistet wird.

An dieser Stelle soll auch angemerkt werden, dass es nicht unbedingt notwendig ist, eine solche Umhüllung bereitzustellen. In manchen Fällen kann es genügen, den Patienten einfach durch ein Auflegen auf eine Vakuummatratze zu immobilisieren, wobei die Vakuummatratze dann beispielsweise an Stellen neben dem Patienten Verankerungen zum Anbringen von Referenzanordnungen aufweist.

Im dargestellten Fall ist der Patient aber im Rumpfbereich umhüllt, und die Figur 2 zeigt einen Schnitt A-A in Figur 1, aus dem deutlich wird, wie die Verankerung 2 innerhalb des Füllmaterials der Umhüllung 1 eingesetzt wird. Auch eine Schließe 3 ist in Figur 2 nochmals zu sehen.

Die Verankerung 2 in der Figur 2 ist eine solche, wie sie auch in Figur 3 zu sehen ist. Sie weist eine Sockelplatte 4 und eine Verbindung bzw. einen Verbindungsabschnitt 9 auf. Die Sockelplatte 4 ist innerhalb des Vakuummatratzen-Füllmaterials eingebettet, wobei der Verbindungsabschnitt 9 durch das Obermaterial der Matratze hindurch nach oben ausragt. Die entstehende Öffnung ist rundum gasdicht abgeschlossen. Durch die Einbettung der Sockelplatte 4 wird diese von dem Matratzen-Füllmaterial von allen Seiten her umschlossen und nach der Evakuierung der Matratze völlig starr fixiert. Diese starre Fixierung der Sockelplatte 4 gegenüber der Matratzenumhüllung 1 und damit auch gegenüber dem Patienten führt letztendlich dazu, dass auch die Referenzanordnung 5 starr und unbeweglich gegenüber denjenigen Patientenkörperteil fixiert wird, die behandelt werden sollen.

Die Referenzanordnung 5 weist an ihrer Oberseite drei Marker auf, beispielsweise reflektierende, passive Marker, die mit Infrarotkameras erfassbar sind. Es können auch Markierungen für sichtbares Licht verwendet werden oder Markierungen, die ihre Position innerhalb von elektrischen oder magnetischen Feldern passiv oder aktiv angeben. Wichtig ist, dass sie von dem verwendeten Navigationssystem geortet werden können.

Die Referenzanordnung 5 wird im vorliegenden Beispiel über eine Anschlusseinrichtung 7 (Adapter) auf den Verbindungsabschnitt 9 des Sockels 2 aufgesetzt, und zwar in starrer und unbeweglicher Weise. Der Adapter. 7 ist nur schematisch dargestellt, hier kann jedwede positionsfeste Steck-, Gewinde-, Schnapp-, oder eine ähnliche Verbindungsart gewählt werden.

Wie wiederum aus Figur 1 erkennbar ist, sind beim vorliegenden Ausführungsbeispiel die Verankerungen 2 um das Fenster 10 herum angeordnet, so dass sie sich in der Nähe des Behandlungsortes befinden werden und damit eine genaue Navigation ermöglichen.

Die vorliegende Erfindung gestattet die stabile und positionstreue Fixierung einer Patientenreferenz gegenüber einem Patienten, ohne dass hierzu ein invasiver Eingriff notwendig wird. Besonders im spinalen Anwendungsbereich können demnach mit der vorliegenden Erfindung und unter sehr genauer Navigationsunterstützung Operationen minimal invasiv durchgeführt werden, z.B. die Verabreichung von Spritzen in den Zwischenwirbelbereich oder die Anwendung der Vertebroplastie, wo Kanülen positioniert werden müssen, um Stützmassen in Wirbelkörper einzubringen. Natürlich ist die Erfindung aber auch für alle anderen Anwendungen geeignet, die minimal invasiv ablaufen sollen.

Abschließend ist noch anzumerken, dass die Basisregistrierung einer erfindungsgemäß angebrachten Referenzanordnung im Navigationssystem bei der intraoperativen Anwendung der Erfindung vorteilhafterweise berührungslos geschehen wird. Dabei ist es von Vorteil, wenn für das Matratzen- und Füllmaterial ein Material verwendet wird, das für Röntgenstrahlen durchlässig ist, da in solchen Fällen eine berührungslose Basisregistrierung über Rönten-C-Bögen, Navigationssysteme mit Fluoro zu CT bzw. Fluoro zu MR Registrierung oder über 3D-C-Bögen geschehen kann, die dreidimensionale Bilder aus mehreren Einzelaufnahmen erstellen.

## Patentansprüche

1. Fixierungssystem zum Fixieren einer Navigations-Referenzanordnung (5) für die bildunterstützte medizinische Behandlung gegenüber einem Patienten, mit einer Immobilisierungseinrichtung (1), mittels der eine Referenzanordnung (5) außerhalb des Patientenkörpers angeordnet werden kann, **dadurch gekennzeichnet, dass** die Immobilisierungseinrichtung eine Vakuummatratze oder eine Vakuummatratzen-Patientenumhüllung (1) ist und an oder in der Vakuummatratze bzw. Vakuummatratzen-Patientenumhüllung (1) mindestens eine Verankerung (2) für die Referenzanordnung (5) vorgesehen ist, wobei mittels der Verankerung (2) die Referenzanordnung (5) direkt an der Vakuummatratze bzw. Vakuummatratzen-Patientenumhüllung (1) selbst angebracht werden kann.

2. Fixierungssystem nach Anspruch 1, **dadurch gekennzeichnet, dass** die Verankerung (2) einen Sockel bzw. eine Sockelplatte (4) aufweist, der bzw. die bei der Evakuierung der Vakuummatratze oder der Vakuummatratzen-Patientenumhüllung (1) gegenüber dieser in einer festen Position starr fixiert wird.

3. Fixierungssystem nach Anspruch 2, **dadurch gekennzeichnet, dass** der Sockel bzw. die Sockelplatte (4) innerhalb des Füllmaterials der Vakuummatratze bzw. Vakuummatratzen-Patientenumhüllung (1) eingebettet ist und eine Verbindung (9) zur Referenzanordnung (5) aufweist, die durch eine gasdicht abgeschlossene Öffnung zur Außenseite ragt.

4. Fixierungssystem nach Anspruch 3, **dadurch gekennzeichnet, dass** die Referenzanordnung (5) einteilig mit der Verbindung (9) und dem Sockel bzw. der Sockelplatte (4) ausgebildet ist.

5. Fixierungssystem nach Anspruch 3, **dadurch gekennzeichnet, dass** die Referenzanordnung als separates Bauteil ausgebildet ist und über einen Adapter (7) auf die Verbindung (9) gesetzt werden kann.

6. Fixierungssystem nach einem der Ansprüche 1 bis 5, **dadurch gekennzeichnet, dass** mehrere Verankerungen an beabstandeten Stellen der Vakuummatratze bzw. der Vakuummatratzen-Patientenumhüllung vorgesehen sind.

7. Fixierungssystem nach einem der Ansprüche 1 bis 6, **dadurch gekennzeichnet, dass** die Vakuummatratze bzw. die Vakuummatratzen-Patientenumhüllung (1) mindestens eine Aussparung bzw. ein Durchgangsfenster (10) aufweist, das den Zugang zu behandlungsbedürftigen Patientenkörperteilen gestattet.

8. Fixierungssystem nach Anspruch 7, **dadurch gekennzeichnet, dass** die Verankerungen (2) um die Peripherie des Durchgangsfensters angeordnet sind.

9. Fixierungssystem nach einem der Ansprüche 1 bis 8, **dadurch gekennzeichnet, dass** die Vakuummatratzen-Patientenumhüllung (1) so bemessen ist, dass sie den Rumpfbereich des Patienten umhüllen kann, wobei sie mit Hilfe von Schließen (3) an ihren Randbereichen vorfixiert werden kann.

## Claims

1. A fixing system for fixing a navigational reference array (5) for image-assisted medical treatment with respect to a patient, comprising an immobilising means (1) by means of which a reference array (5) can be arranged outside the patient's body, **characterised in that** said immobilising means is a vacuum mattress or a vacuum mattress patient jacket (1) and at least one anchoring (2) for the reference array (5) is provided on or in said vacuum mattress or vacuum mattress patient jacket (1), wherein the reference array (5) can be attached directly to the vacuum mattress or vacuum mattress patient jacket (1) itself by means of the anchoring (2).

2. The fixing system as set forth in claim 1, **characterised in that** said anchoring (2) comprises a base or base plate (4) which is rigidly fixed in a fixed position with respect to the vacuum mattress or vacuum mattress patient jacket (1), when the latter is evacuated.

3. The fixing system as set forth in claim 2, **characterised in that** said base or base plate (4) is embedded within the filling material of the vacuum mattress or vacuum mattress patient jacket (1) and comprises a connection (9) to the reference array (5) which protrudes to the outer side through an opening which is sealed gas-tight.

4. The fixing system as set forth in claim 3, **characterised in that** the reference array (5) is formed in one part together with said connection (9) and the base or base plate (4).

5. The fixing system as set forth in claim 3, **characterised in that** the reference array is formed as a separate component and can be placed onto the connection (9) via an adaptor (7).

6. The fixing system as set forth in any one of claims 1 to 5, **characterised in that** a number of anchorings are provided at spaced locations on the vacuum mattress or vacuum mattress patient jacket.

7. The fixing system as set forth in any one of claims 1 to 6, **characterised in that** the vacuum mattress or vacuum mattress patient jacket (1) comprises at least one recess or transit window (10) which allows access to parts of the patient's body in need of treatment.

8. The fixing system as set forth in claim 7, **characterised in that** the anchorings (2) are arranged around the periphery of the transit window.

9. The fixing system as set forth in any one of claims 1 to 8, **characterised in that** the vacuum mattress patient jacket (1) is dimensioned such that it can envelope the torso area of the patient, wherein it can be pre-fixed with the aid of clasps (3) on its peripheral areas.

## Revendications

1. Système de fixation pour fixer une structure de référence de navigation (5) pour le traitement médical assisté par imagerie, par rapport à un patient, comportant un dispositif d'immobilisation (1) au moyen duquel on peut disposer une structure de référence (5) à l'extérieur du corps du patient, **caractérisé en ce que** le dispositif d'immobilisation est un matelas sous vide ou une enveloppe de patient à matelas sous vide (1), et sur ou dans le matelas sous vide ou l'enveloppe de patient à matelas sous vide (1) est prévu au moins un ancrage (2) pour la structure de référence (5), la structure de référence (5) pouvant être placée , il au moyen de l'ancrage (2), directement sur le matelas sous vide ou l'enveloppe de patient à matelas sous vide (1) elle-même.

2. Système de fixation selon la revendication 1, **caractérisé en ce que** l'ancrage (2) comporte un socle ou une plaque formant socle (4) qui est fixée rigidement dans une position par rapport au matelas sous vide ou à l'enveloppe de patient à matelas sous vide (1), lors de leur mise sous vide.

3. Système de fixation selon la revendication 2, **caractérisé en ce que** le socle ou la plaque formant socle (4) est noyée à l'intérieur du matériau de garniture du matelas sous vide ou de l'enveloppe de patient à matelas sous vide (1), et présente une liaison (9) avec la structure de référence (5), qui passe à travers une ouverture, fermée de manière étanche aux gaz, vers le côté extérieur.

4. Système de fixation selon la revendication 3, **caractérisé en ce que** la structure de référence (5) est réalisée d'une seule pièce avec la liaison (9) et le socle ou la plaque formant socle (4).

5. Système de fixation selon la revendication 3, **caractérisé en ce que** la structure de référence est réalisée sous la forme d'un composant séparé et peut être placée sur la liaison (9), par l'intermédiaire d'un adaptateur (7).

6. Système de fixation selon l'une des revendications 1 à 5, **caractérisé en ce que** plusieurs ancrages sont prévus en des emplacements espacés du matelas sous vide ou de l'enveloppe de patient à matelas sous vide.

7. Système de fixation selon l'une des revendications 1 à 6, **caractérisé en ce que** le matelas sous vide ou l'enveloppe de patient à matelas sous vide (1) présente au moins une découpe ou une fenêtre de passage (10) qui permet l'accès aux parties à traiter du corps du patient.

8. Système de fixation selon la revendication 7, **caractérisé en ce que** les ancrages (2) sont disposés autour de la périphérie de la fenêtre de passage.

9. Système de fixation selon l'une des revendications 1 à 8, **caractérisé en ce que** l'enveloppe de patient à matelas sous vide (1) a des dimensions telles qu'elle peut envelopper la région du buste du patient, et peut être fixée sur ses zones de bordure à l'aide de boucles (3).
